(19) Europäisches Patentamt

European Patent Office

Office européen des brevets

(11) **EP 1 254 076 B1**

(12) **FASCICULE DE BREVET EUROPEEN**

(45) Date de publication et mention
de la délivrance du brevet:
**27.04.2005 Bulletin 2005/17**

(51) Int Cl.⁷: **C01G 43/025**, G21C 3/62,
G01N 33/00

(21) Numéro de dépôt: **01907757.7**

(22) Date de dépôt: **09.02.2001**

(86) Numéro de dépôt international:
**PCT/FR2001/000391**

(87) Numéro de publication internationale:
**WO 2001/058810 (16.08.2001 Gazette 2001/33)**

(54) **PROCEDE ET DISPOSITIF DE DETERMINATION DE L'EVOLUTION D'UNE REACTION CHIMIQUE DANS UN FOUR ET DE REGLAGE DE LA REACTION**

VERFAHREN UND VORRICHTUNG ZUR BESTIMMUNG DER ENTWICKLUNG EINER CHEMISCHEN REAKTION IN EINEM OFEN UND ZUR STEUERUNG DER REAKTION

METHOD AND DEVICE FOR DETERMINING THE EVOLUTION OF A CHEMICAL REACTION IN A KILN AND FOR ADJUSTING THE REACTION

(84) Etats contractants désignés:
**FR GB SE**

(30) Priorité: **11.02.2000 FR 0001756**

(43) Date de publication de la demande:
**06.11.2002 Bulletin 2002/45**

(73) Titulaire: **Société Franco-Belge de Fabrication de Combustibles - FBFC**
**92400 Courbevoie (FR)**

(72) Inventeur: **FEUGIER, André**
**F-26300 Chatuzange le Goubet (FR)**

(74) Mandataire: **Bouget, Lucien et al**
**Cabinet Lavoix**
**2, Place d'Estienne d'Orves**
**75441 Paris Cédex 09 (FR)**

(56) Documents cités:
EP-A- 0 322 481          FR-A- 2 771 725
US-A- 3 765 844          US-A- 5 723 100

- **PATENT ABSTRACTS OF JAPAN vol. 1998, no. 13, 30 novembre 1998 (1998-11-30) & JP 10 227881 A (JAPAN NUCLEAR FUEL CO LTD&LT;JNF&GT;), 25 août 1998 (1998-08-25)**
- **PATENT ABSTRACTS OF JAPAN vol. 1999, no. 01, 29 janvier 1999 (1999-01-29) & JP 10 279311 A (MITSUBISHI MATERIALS CORP), 20 octobre 1998 (1998-10-20)**
- **DATABASE WPI Week 198709 Derwent Publications Ltd., London, GB; AN 1987-061716[09] XP002154513 & JP 62 017694 A (TOSHIBA KK), 26 janvier 1987 (1987-01-26)**

**Description**

L'invention concerne un procédé et un dispositif de détermination de l'avancement d'une réaction chimique dans un four et de réglage de la réaction. En particulier, l'invention s'applique à la production d'une poudre de dioxyde d'uranium utilisée pour la fabrication de pastilles de combustible nucléaire.

Dans les réacteurs nucléaires, par exemple dans les réacteurs nucléaires à eau sous pression, on utilise un combustible qui peut être constitué principalement par de l'oxyde d'uranium ou par un mélange d'oxyde d'uranium et de plutonium.

Le combustible nucléaire, qui est enrichi en éléments fissiles, par exemple de l'uranium 235 dans le cas d'un combustible constitué d'oxyde d'uranium, est généralement obtenu par un procédé dans lequel le produit final de l'enrichissement est constitué par de l'hexafluorure d'uranium $UF_6$ gazeux.

On réalise ensuite la conversion de l'$UF_6$ en oxyde d'uranium par oxydation, par exemple en utilisant de la vapeur d'eau.

Les procédés utilisés actuellement qui donnent les meilleurs résultats pour la conversion de l'hexafluorure d'uranium en oxyde d'uranium sont les procédés de conversion directe par voie sèche qui sont mis en oeuvre dans des dispositifs comportant successivement un réacteur muni de moyens d'introduction d'$UF_6$ et de vapeur d'eau dans la chambre du réacteur dans laquelle on réalise la formation d'oxyfluorure d'uranium $UO_2F_2$ à partir de l'$UF_6$ et un four tubulaire tournant dans lequel on transforme l'oxyfluorure d'uranium $UO_2F_2$ solide pulvérulent en oxyde d'uranium, le four tubulaire étant muni de moyens de chauffage et de moyens d'introduction de vapeur d'eau et d'hydrogène, par la partie de sortie du four tournant à contre-courant du solide pulvérulent circulant dans la direction longitudinale du four.

On récupère à la sortie du four tournant de la poudre d'oxyde d'uranium constituée principalement de dioxyde $UO_2$, cette poudre étant ensuite conditionnée dans une installation de conditionnement, avant d'être utilisée pour la fabrication de pastilles frittées de combustible nucléaire.

Le procédé de transformation de l'oxyfluorure d'uranium $UO_2F_2$ en oxyde d'uranium est réalisé par mise en contact d'un solide sous forme pulvérulente avec un mélange de gaz réactif contenant en particulier de la vapeur d'eau et de l'hydrogène.

Il se forme dans le four tournant de l'acide fluorhydrique HF par réaction d'oxydation de l'oxyfluorure d'uranium par la vapeur d'eau.

La matière solide en circulation dans le four tournant venant en contact avec le mélange de gaz réactif est le siège de différentes réactions chimiques qui conduisent à la formation d'oxyde d'uranium et principalement de dioxyde $UO_2$ et en particulier les réactions chimiques indiquées ci-dessous :

$$UO_2F_2 + H_2O \rightarrow UO_3 + 2HF$$

$$3UO_3 \rightarrow U_3O_8 + \tfrac{1}{2} O_2$$

$$U_3O_8 + 2H_2 \rightarrow 3UO_2 + 2H_2O.$$

La composition du produit solide en circulation continue dans le four et la composition du mélange gazeux réactif sont donc essentiellement variables dans la direction longitudinale du four, suivant laquelle se produisent la circulation de la matière solide pulvérulente et la circulation du mélange gazeux, à contre-courant.

De manière à contrôler le mieux possible les réactions chimiques dans le four, on réalise un réglage d'éléments de chauffage du four disposés à la périphérie de l'enveloppe du four tournant, de manière à obtenir une répartition réglée de la température suivant la direction longitudinale du four (FR-A-2.771.725).

Ce procédé de réglage de la température suivant la direction longitudinale axiale du four ne permet cependant pas de maîtriser efficacement la composition des oxydes d'uranium à la sortie du four.

Le réglage des débits d'hydrogène et de vapeur d'eau introduits par la partie d'extrémité de sortie du four tournant ne permet pas d'améliorer le contrôle des réactions de conversion dans le four, du fait de la dilution des gaz réactifs dans le four et du caractère aléatoire de la répartition des gaz réactifs obtenue à l'intérieur de l'enceinte du four.

En outre, la formation de dioxyde d'uranium $UO_2$ à partir de l'oxyde $U_3O_8$ peut se produire par des réactions intermédiaires au cours desquelles on obtient différents oxydes d'uranium, suivant la chaîne de transformation

$$U_3O_8 \rightarrow U_3O_7 \rightarrow U_4O_9 \rightarrow UO_2.$$

De manière générale, on ne connaissait pas de procédé permettant de déterminer l'avancement des réactions entre l'oxyfluorure ou les oxydes d'uranium en circulation dans le four tournant et le mélange gazeux réactif, suivant la direction longitudinale de circulation des produits dans le four tournant. La connaissance d'une courbe d'évolution des réactions à l'intérieur du four tournant permettrait d'influer sur les réactions de manière à optimiser le processus de conversion pour obtenir, en sortie du four, des oxydes de composition voulue.

En particulier, pour obtenir par compression, à partir de la poudre d'oxyde d'uranium, des pastilles crues ayant une très bonne résistance mécanique mesurée par des essais d'écrasement, de microdureté ou d'attrition, on a observé qu'il était nécessaire d'utiliser des poudres d'oxyde dont la composition est telle que le rapport atomique de l'oxygène et de l'uranium O/U soit nettement plus élevé que les rapports habituellement obtenus dans le cas des oxydes en sortie d'un four de conversion de l'oxyfluorure d'uranium, ces oxydes étant constitués principalement par du dioxyde d'uranium $UO_2$.

Pour augmenter le rapport O/U, on a par exemple proposé de mélanger, dans certaines proportions, des particules de dioxyde d'uranium $UO_2$ obtenues par conversion par voie sèche et des particules d'un oxyde tel que $U_3O_8$. Ce procédé, permettant d'augmenter le rapport O/U des oxydes utilisés pour fabriquer des pastilles de matériau combustible, nécessite d'oxyder de l'oxyde d'uranium $UO_2$ dans des conditions parfaitement réglées, pour obtenir l'oxyde $U_3O_8$ puis de réaliser un mélange homogène de particules d'$UO_2$ et de $U_3O_8$. Le procédé d'obtention des poudres d'oxyde d'uranium est donc complexe.

On ne connaissait pas jusqu'ici de procédé permettant, à partir d'une détermination précise de l'avancement de la réaction chimique de conversion dans le four, de régler la réaction, de manière à obtenir des oxydes d'uranium en sortie du four, de composition voulue, et en particulier des oxydes d'uranium ayant un rapport O/U élevé, c'est-à-dire des oxydes ayant une formule moyenne du type $UO_{2+x}$, avec x relativement élevé (x compris entre 0,03 et 0,7 et de préférence entre 0,05 et 0,25).

De manière plus générale, dans le cas où l'on effectue une réaction chimique entre un produit dans un état condensé, par exemple un produit solide pulvérulent circulant en continu dans une direction longitudinale d'un four et un mélange gazeux réactif, on ne connaissait pas de procédé pour déterminer de manière précise l'état d'avancement de la réaction en différents points suivant la direction longitudinale du four et de procédé de réglage de la réaction dans le four à partir de cette détermination précise.

Une telle détermination précise de l'état d'avancement de la réaction dans le four doit être mise en oeuvre sans ouverture du four et sans risque d'introduction d'air à l'intérieur du four, ce qui, d'une part, fausserait totalement les mesures et analyses effectuées et, d'autre part, produirait des modifications du produit en cours de fabrication dans le four. Il n'est donc pas possible de contrôler l'état d'avancement des réactions en effectuant des prélèvements du produit pulvérulent en circulation à différentes abscisses suivant l'axe et à l'intérieur de l'enceinte du four.

Le but de l'invention est donc de proposer un procédé de détermination de l'évolution de la conversion d'oxyfluorure d'uranium sous forme pulvérulente en circulation dans un four tournant, en contact avec un mélange gazeux réactif réalisant la conversion de l'oxyflurorure d'uranium en oxydes d'uranium sous forme pulvérulente par des réactions chimiques se produisant pendant la circulation des produits sous forme pulvérulente à l'intérieur du four, de sorte que la composition des produits sous forme pulvérulente et la composition du mélange gazeux renfermant en particulier $H_2O$, $H_2$ et HF, en circulation dans le four évoluent suivant la direction longitudinale du four, ce procédé permettant de déterminer l'état d'avancement et l'évolution de la réaction chimique dans la direction longitudinale du four, de manière exacte et précise, sans qu'il soit nécessaire d'ouvrir le four et sans modifier l'évolution de la réaction chimique, du fait de la mise en oeuvre du procédé.

Dans ce but, on prélève une pluralité d'échantillons du mélange gazeux chacun en un point d'une pluralité de points de référence espacés les uns des autres suivant la direction longitudinale du four pour déterminer la composition du mélange gazeux, on déduit de la composition du mélange gazeux en chacun des points de référence le degré d'avancement des réactions chimiques de formation des oxydes d'uranium dans le four tournant, suivant sa direction longitudinale, et on réalise une modélisation du fonctionnement du four tournant, sous la forme de courbes d'évolution des réactions chimiques de formation des oxydes d'uranium suivant la direction longitudinale du four tournant.

L'invention est également relative à un procédé de réglage d'au moins une réaction chimique dans un four, pour obtenir un produit final de composition déterminée, en utilisant une détermination préalable de l'évolution de la réaction chimique dans la direction longitudinale du four permettant de modéliser le fonctionnement du four. Le réglage de la réaction chimique est alors effectué en injectant des gaz de composition soigneusement choisie en au moins un point à l'intérieur de l'enceinte du four, avec un débit prédéterminé.

Afin de bien faire comprendre l'invention, on va décrire à titre d'exemple en se référant aux figures jointes en annexe, la mise en oeuvre du procédé de détermination et du procédé de réglage suivant l'invention, dans le cas d'un four tournant de conversion d'oxyfluorure d'uranium en oxyde d'uranium.

La figure 1 est une vue en coupe longitudinale d'une installation de conversion d'hexafluorure d'uranium en oxyde d'uranium, comportant un four tournant équipé d'un dispositif de prélèvement et d'injection permettant de mettre en oeuvre les procédés suivant l'invention.

La figure 2 est une vue en perspective du dispositif de prélèvement et d'injection selon l'invention.

La figure 3 est une vue d'une partie centrale de support d'une canne de prélèvement du dispositif représenté sur la figure 2.

La figure 4 est une vue de face d'une bride de fixation du dispositif de prélèvement et d'injection représenté sur la figure 2.

La figure 5 est une vue en coupe suivant 5-5 de la figure 4.

La figure 6 est une vue en coupe axiale d'un ensemble de prélèvement et d'injection du dispositif représenté sur la figure 2.

Les figures 7A, 7B et 7C sont des vues schématiques dans trois positions fonctionnelles différentes d'un circuit d'analyse et de purge disposé à l'extérieur du four tournant et relié au dispositif de prélèvement et d'injection suivant l'invention.

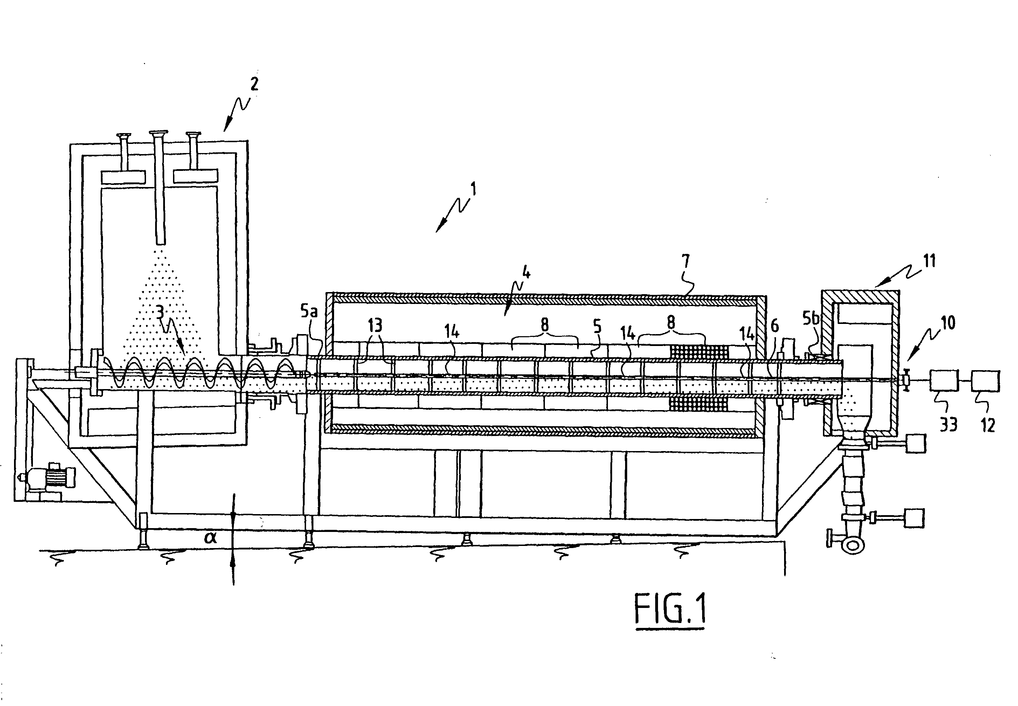

Sur la figure 1, on voit une installation de conversion d'hexafluorure d'uranium en oxyde d'uranium, désignée de manière générale par le repère 1, et qui comporte un réacteur 2 de conversion de l'hexafluorure d'uranium $UF_6$ en oxyfluorure d'uranium $UO_2F_2$, par injection dans le réacteur 2 d'hexaflurorure gazeux $UF_6$, de vapeur d'eau et d'azote.

L'hexafluorure d'uranium $UF_6$ est transformé en oxyfluorure $UO_2F_2$ par oxydation sous l'effet de la vapeur selon la réaction :

$$UF_6 + 2H_2O \rightarrow UO_2F_2 + 4\ HF.$$

L'oxyfluorure d'uranium $UO_2F_2$ résultant de la réaction est produit sous forme de poudres qui tombent dans le fond du réacteur où ces poudres sont reprises par une vis de transport 3 pour être introduites dans la partie d'entrée d'un four rotatif 4 à l'intérieur duquel on réalise la conversion des poudres d'oxyfluorure d'uranium en oxyde d'uranium.

Le four 4 comporte une enveloppe tubulaire 5 montée tournante et entraînée en rotation autour de son axe longitudinal 6 qui est incliné d'un angle $\alpha$ par rapport au plan horizontal, de manière que l'extrémité d'entrée 5a de l'enveloppe tournante du four soit disposée à un niveau supérieur au niveau de l'extrémité de sortie 5b.

Autour de l'enveloppe tournante du four, à l'intérieur d'une enceinte calorifuge 7, sont disposés des éléments de chauffage 8 permettant de chauffer le volume intérieur de l'enveloppe tournante 5 dans laquelle on réalise la transformation à haute température de l'oxyfluorure d'uranium en oxyde d'uranium suivant les réactions indiquées ci-dessous :

$$UO_2F_2 + H_2O \rightarrow UO_3 + 2\ HF \qquad (1)$$

$$3\ UO_3 \rightarrow U_3O_8 + \tfrac{1}{2}\ O_2 \qquad (2)$$

$$U_3O_8 + 2\ H_2 \rightarrow 3\ UO_2 + 2\ H_2O \qquad (3)$$

$$\tfrac{1}{2}\ O_2 + H_2 \rightarrow H_2O \qquad (4)$$

$$U_3O_8 \rightarrow U_3O_7 \rightarrow U_4O_9 \rightarrow UO_2 \qquad (5)$$

La vapeur d'eau et l'hydrogène nécessaires pour réaliser la conversion à l'intérieur du four tournant sont introduits dans le volume interne de l'enveloppe tournante 5, généralement par un moyen d'injection introduit par l'extrémité de sortie 5b de l'enveloppe tournante 5 du four.

La poudre d'oxyfluorure d'uranium $UO_2F_2$ introduite par la vis 3 dans l'extrémité d'entrée 5a de l'enveloppe tournante 5 est transportée vers l'extrémité de sortie 5b, lors de la rotation du four tournant, du fait de la pente $\alpha$ de l'axe longitudinal de cette enveloppe 5. De plus, la rotation du four produit une agitation et un soulèvement des poudres qui viennent ainsi en contact intime avec la vapeur d'eau et l'hydrogène injectés dans l'enveloppe du four qui circulent généralement à contre-courant de la circulation du matériau pulvérulent à l'intérieur de l'enveloppe tournante.

L'extrémité 5b de l'enveloppe tournante 5 débouche à l'intérieur d'une chambre d'une installation 11 de récupération et de conditionnement de la poudre d'oxyde d'uranium formée dans le four par conversion de la poudre d'oxyfluorure d'uranium.

Les différents gaz formés à l'intérieur de l'enceinte du four par les réactions de transformation de l'oxyfluorure et des oxydes d'uranium et en particulier l'acide fluorhydrique HF sont récupérés avec l'hydrogène et la vapeur d'eau excédentaires qui n'ont pas été utilisés pour la mise en oeuvre des réactions chimiques dans le four.

Comme expliqué plus haut, l'un des problèmes inhérents à la conduite d'un procédé de conversion de type conversion par voie sèche mis en oeuvre dans une installation telle que l'installation 1 résulte du fait qu'on ne dispose généralement pas d'information sur le déroulement de la conversion à l'intérieur de l'enveloppe du four et sur l'état d'avancement suivant la direction axiale 6 du four tournant des différentes réactions mises en oeuvre.

Une bonne connaissance de l'évolution des réactions chimiques à l'intérieur du four tournant permet, comme il sera expliqué plus loin, de régler les réactions par injection de gaz réactifs et en particulier d'hydrogène à l'intérieur du four tournant, en des emplacements déterminés, afin d'obtenir, en sortie du four, un mélange d'oxyde d'uranium de composition générale $UO_{2+x}$, avec x fixé à une valeur élevée prédéterminée, c'est-à-dire avec un rapport O/U largement supérieur à 2 et fixé à une valeur bien déterminée.

Différents moyens ont permis jusqu'ici d'influer sur les réactions chimiques à l'intérieur du four et en particulier la commande des éléments de chauffage pour obtenir une répartition de température optimisée à l'intérieur de l'enveloppe de four, suivant la direction longitudinale 6 et l'utilisation de chicanes 13 fixées à l'intérieur du four pour favoriser le contact entre le mélange gazeux réactif circulant dans le four et le produit pulvérulent en cours de conversion.

Un moyen supplémentaire de réglage des réactions chimiques à l'intérieur du four tournant pourrait consister à régler les débits d'hydrogène et de vapeur d'eau introduits par l'extrémité de sortie 5b de l'enveloppe tournante, généralement par l'intermédiaire d'une chambre fixe de l'installation de récupération et de conditionnement de la poudre d'oxyde d'uranium. Cependant, on n'a jamais pu obtenir un réglage satisfaisant des oxydes d'uranium produits en réglant les débits de vapeur d'eau et d'hydrogène introduits par l'extrémité de sortie du four.

Selon l'invention, on réalise une détermination précise de l'évolution des réactions chimiques à l'intérieur du four en utilisant une canne 10 de prélèvement de gaz en différents points répartis suivant la direction longitudinale 6 du four reliée à un analyseur de gaz 12, à l'extérieur du four.

En effet, l'état d'avancement des différentes réactions mises en oeuvre à l'intérieur du four tournant qui ont été rappelées ci-dessus peut être déterminé, en chacun des points du four, par une mesure de la proportion ou de la pression partielle de gaz produits par les différentes réactions ou intervenant dans ces réactions tels qu'en particulier HF, $H_2$ et $H_2O$. En particulier, la présence ou l'absence d'un gaz en un point de référence suivant la direction longitudinale 6 du four peut permettre de savoir si une réaction chimique de conversion est en cours ou a été achevée.

Avant la mise en service d'une installation de conversion telle que l'installation 1, on réalise une première opération de modélisation qui consiste à faire fonctionner l'installation dans ses conditions normales de production et à prélever, en une pluralité de points de prélèvement répartis suivant la direction longitudinale 6 du four tournant, les gaz présents dans le four, puis à analyser ces gaz à l'extérieur du four.

Les résultats des analyses des gaz en différents points du four sont utilisés pour construire un modèle traduisant l'évolution des différentes réactions chimiques mises en oeuvre suivant la longueur du four.

Après cette première phase de modélisation du fonctionnement du four tournant, on détermine, à partir des résultats de la modélisation, les injections nécessaires en différents points du four, en particulier des injections d'hydrogène pour obtenir, en sortie du four, une poudre $UO_{2+x}$ ayant un rapport O/U optimisé. Généralement, on vise un rapport O/U compris entre 2 et 2,7 et de préférence entre 2,10 et 2,25.

On peut envisager de produire des oxydes avec un rapport O/U aussi élevé que 2,66, ce qui n'était possible jusqu'ici qu'en réalisant un mélange des oxydes $UO_2$ et $UO_3O_8$.

Pour réaliser le prélèvement et l'analyse des gaz à l'intérieur du four (ainsi qu'éventuellement l'injection de gaz dans la phase de production du four), on utilise une canne 10 de grande longueur, dont la longueur est au moins égale à la longueur axiale de l'enveloppe 5 du four tournant et qui est introduite dans le volume intérieur de l'enveloppe tournante 5 par son extrémité de sortie 5b et disposée suivant sa direction axiale longitudinale 6. La canne 10 est fixée par une partie d'extrémité extérieure à l'enveloppe tournante du four à une extrémité du four, sur une partie fixe telle que la paroi de la chambre de récupération de l'installation 11 de récupération et de conditionnement des poudres d'oxyde.

La canne de prélèvement est reliée à son extrémité située à l'extérieur du four et de la chambre de l'installation 11 à un ou plusieurs analyseurs de gaz 12.

La canne 10 disposée suivant la direction axiale longitudinale 6 du four tournant 5 est totalement libre par rapport aux parties tournantes de l'enveloppe 5 et en particulier par rapport aux chicanes 13 occupant une partie de la section transversale de l'enveloppe tournante 5 du four.

Dans le cas du mode de réalisation représenté, la canne de prélèvement 10 comporte dix points de prélèvement 14 répartis suivant sa longueur, de manière à réaliser des prélèvements en dix points de référence du volume intérieur de l'enveloppe tournante 5, avec une distance sensiblement fixe entre deux points de référence successifs.

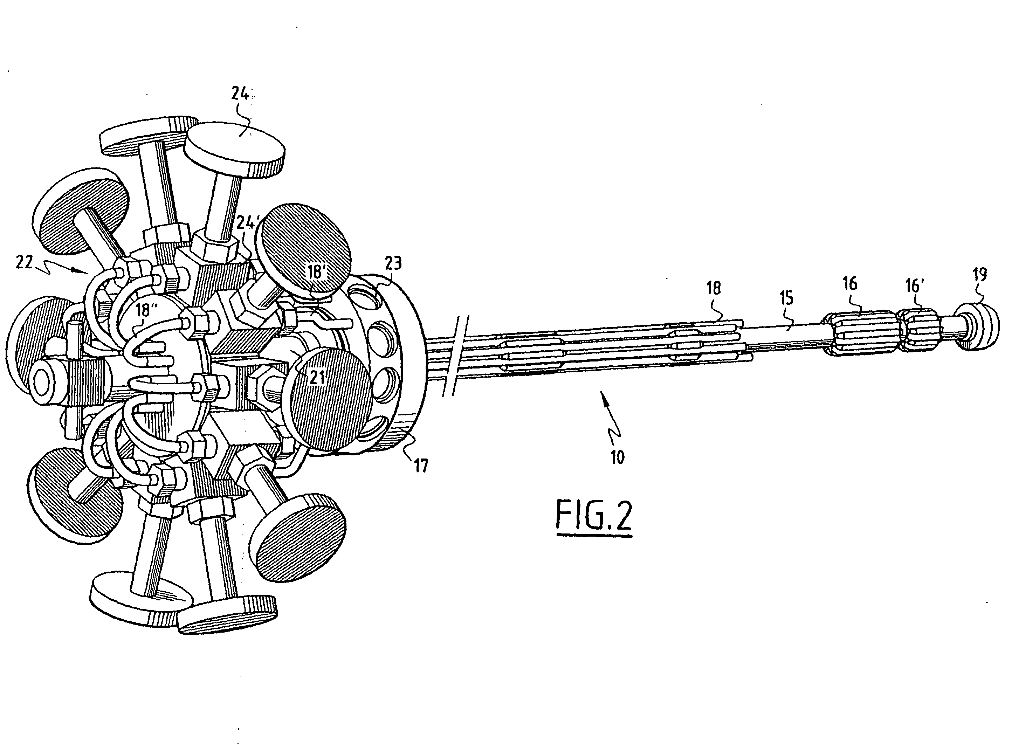

On va maintenant se reporter à la figure 2 pour décrire l'ensemble de la canne de prélèvement 10 suivant l'invention.

La canne de prélèvement 10 comporte un tube central de grande longueur 15 sur lequel sont fixés, de manière coaxiale, avec un espacement prédéterminé dans la direction de l'axe du tube support central 5, des éléments de support et de guidage 16 et 16' de forme globalement cylindrique comportant sur leur surface externe, chacun un ensemble de logements destinés à recevoir chacun un tube de prélèvement 18 d'un ensemble de tubes de prélèvement disposés parallèlement à l'axe longitudinal du tube central 15.

Dans le mode de réalisation représenté, chacun des éléments de guidage et de support 16 et 16' comporte, suivant sa périphérie, dix logements ayant la forme de canaux rectilignes dont la section présente une partie semi-circulaire, usinés dans l'élément de support 16 ou 16'.

Entre deux éléments de support 16, et de manière équidistante par rapport aux deux éléments de support 16, est fixé sur le tube central 15 un élément de support intermédiaire 16', dont la longueur dans la direction axiale est inférieure à la longueur des éléments de support 16.

Les éléments de support 16' comportent une pluralité de logements dont le nombre et la section sont identiques à ceux des logements des éléments de support 16 de plus grande longueur.

Le tube central 15 de la canne 10 est fixé à l'une de ses extrémités suivant l'axe d'une bride 17 de raccordement de la canne 10 à la paroi fixe de l'installation 11, à la sortie du four tournant.

La bride 17 comporte des ouvertures taraudées permettant la fixation de la bride 17 et de la canne 10 sur la paroi de l'installation 11, de telle manière que la canne 10 se trouve dans une position coaxiale par rapport à l'enveloppe 5 du four tournant, c'est-à-dire avec l'axe de son tube central 15 suivant l'axe longitudinal 6 de l'enveloppe 5 du four tournant.

A l'extrémité du tube central 15 opposée à l'extrémité reliée à la bride 17 est fixé un tampon 19 permettant de fermer l'extrémité du tube central 15 qui se trouve engagée à l'intérieur du four, jusque dans une position voisine de l'extrémité d'entrée 5a de l'enveloppe tournante 5.

La bride 17 est solidaire, sur sa face opposée dans la direction axiale à sa face reliée au tube central 15 de la canne, d'un ensemble de prélèvement 22 par l'intermédiaire d'une rallonge 21,.

Chacun des tubes de prélèvement 18 de petit diamètre traverse une partie de la bride 17 dans la direction axiale puis est plié à 90° dans une direction radiale pour être relié, à la périphérie externe de la bride 17, à un élément 23 de raccordement du tube 18 à un prolongateur 18' permettant de mettre en communication le tube 18 avec un corps de vanne 24' d'une vanne 24 du dispositif de prélèvement 22.

Chacun des corps de vanne 24' d'une vanne 24 du dispositif de prélèvement 22 est relié, par un tube de raccordement cintré 18'', à une chambre de prélèvement usinée dans le corps du dispositif de prélèvement 22.

Chacun des tubes 18 peut être mis en communication, par l'intermédiaire des tubes 18' et 18'' et d'une vanne 24, 24', avec la chambre du dispositif de prélèvement 22.

Chacun des tubes 18 s'étend dans la direction axiale de la canne 10 entre la bride 17 et un point de prélèvement 14 correspondant à un point de référence à l'intérieur du four tournant 4, 5, au niveau ou au voisinage d'un élément de support et de guidage 16' de faible longueur.

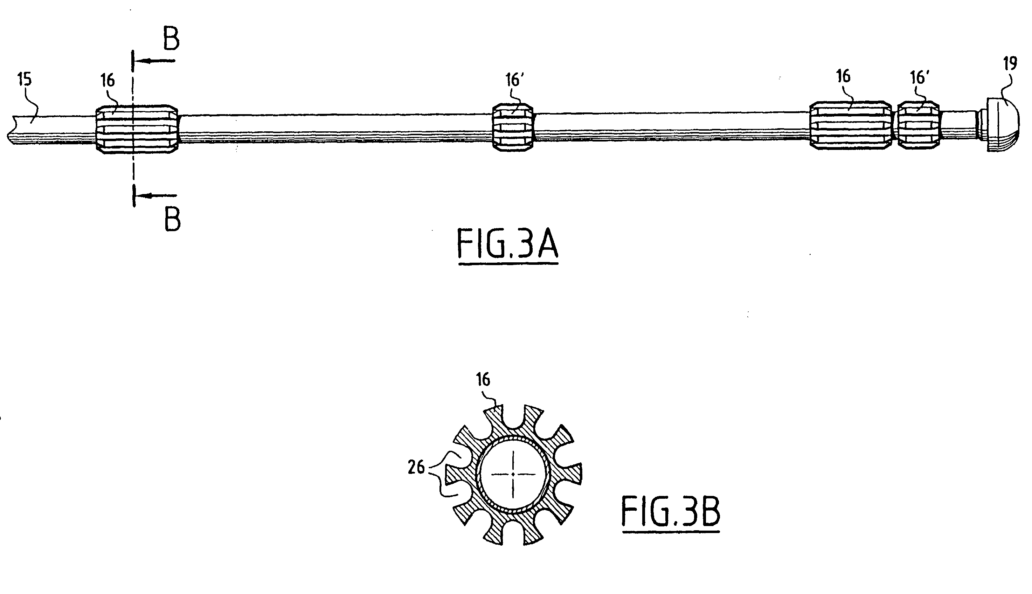

Sur la figure 3A, on a représenté le tube central 15 de la canne de prélèvement 10 sur lequel sont fixés, à intervalles réguliers, des éléments de guidage et de support de tubes 16 de plus grande longueur et, intercalés entre les éléments de support et de guidage 16 de plus grande longueur, des éléments de support et de guidage 16' de plus faible longueur.

Sur la partie d'extrémité du tube central 15 de la canne de prélèvement 10 qui est introduite à l'intérieur de l'enveloppe tournante du four, jusqu'au voisinage de l'extrémité d'entrée de l'enveloppe tournante, est fixé, à la suite du dernier élément de guidage 16 de plus grande longueur, un élément de guidage 16' de plus faible longueur destiné à recevoir le dernier tube 18 effectuant le prélèvement au dernier point de prélèvement 14 situé vers l'extrémité d'entrée 5b de l'enveloppe tournante du four.

La partie d'extrémité du tube central 15 opposée à l'extrémité située vers la partie d'entrée de l'enveloppe tournante du four ne comporte pas d'éléments de support et de guidage 16 ou 16', suivant un tronçon dont l'extrémité est destinée à recevoir la bride 17 de fixation de la canne de prélèvement sur la structure fixe de l'installation.

La partie d'extrémité de la canne comportant le tronçon d'extrémité du tube central 15 dépourvu d'éléments de guidage 16 et 16' est engagée à travers la chambre de l'installation de récupération et de conditionnement de poudres 11 et dans la partie de sortie de l'enveloppe tournante 5, lorsque la canne de prélèvement 10 est fixée en position de service. Dans cette partie de la canne de prélèvement, les tubes de prélèvement 18 placés à la périphérie du tube central 15 ne sont pas fixés sur la paroi externe du tube central 15.

Sur la figure 3B on a représenté la section transversale d'un élément de guidage et de support 16 qui comporte dix logements 26 destinés à recevoir chacun un tube de prélèvement 18 et réalisés sous la forme de canaux dont le fond présente une forme semi-circulaire, s'étendant dans la direction axiale sur la surface périphérique de l'élément de support et de guidage 16 de forme globalement cylindrique.

La section des éléments de guidage et de support 16' de faible longueur est identique à la section d'un élément de guidage et de support de plus grande longueur 16 représentée sur la figure 3B.

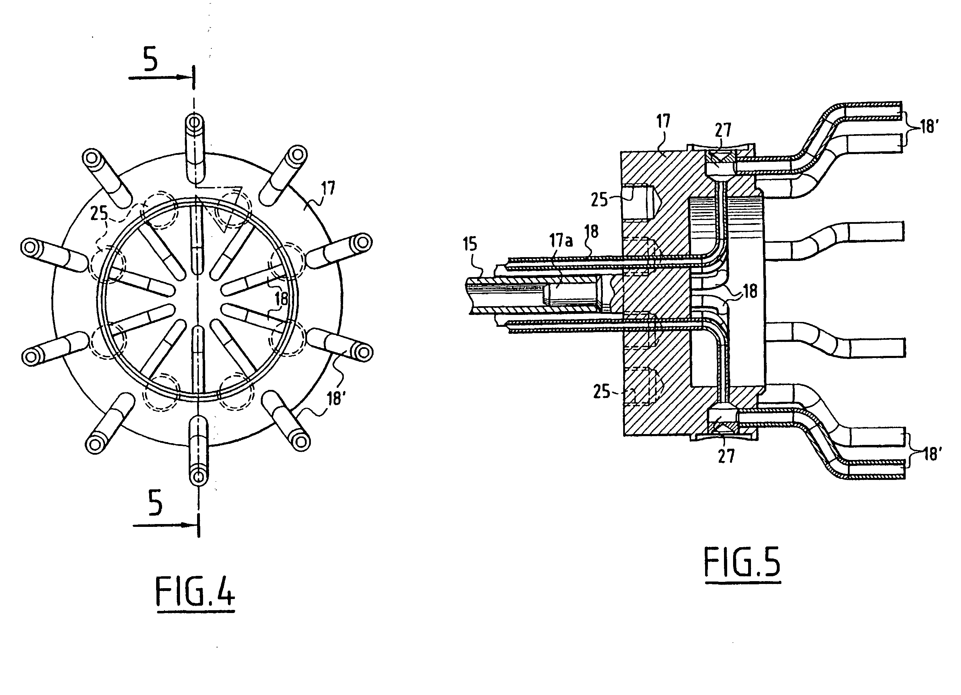

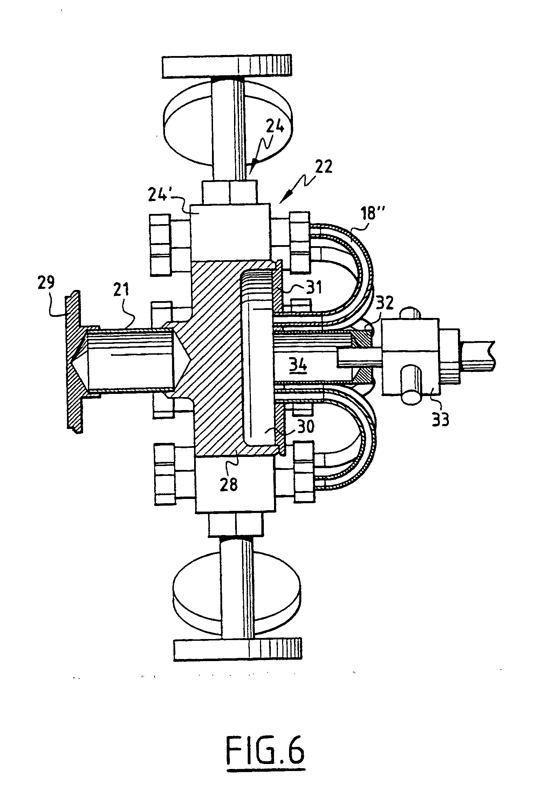

Sur la figure 4 et sur la figure 5, on voit la bride 17 sur laquelle est fixée une des extrémités du tube central 15 de la canne 10 et qui assure le raccordement entre les secondes parties d'extrémité des tubes de prélèvement 18 et les prolongements 18' de chacun de ces tubes de prélèvement.

La bride 17 comporte, sur l'une de ses faces, dans la direction axiale, une partie en saillie 17a sur laquelle est engagée et fixée par soudure l'extrémité du tube central 15.

Les tubes de prélèvement 18 disposés autour du tube central sont engagés chacun dans une ouverture traversante de direction axiale, de la bride 17 débouchant dans une cavité cylindrique sur la seconde face de la bride 17.

Les ouvertures traversantes de passage des tubes 18 à travers la bride 17 sont disposées sous la forme d'une rangée circulaire dans la partie centrale de la bride 17 autour de la partie en saillie 17a.

Dans la partie périphérique externe de la bride 17 sont réalisées des ouvertures taraudées 25 permettant la fixation de la bride 17 sur une partie fixe de l'installation de conversion, par exemple sur la paroi de la chambre du dispositif de récupération et de conditionnement des pulvérulents.

La partie d'extrémité des tubes de prélèvement 18 est cintrée à 90° de manière à être placée dans une direction radiale par rapport à la bride 17 et engagée dans une ouverture de la bride 17 débouchant dans une chambre 27 de raccordement de l'extrémité du tube de prélèvement 18 avec un prolongement 18' cintré présentant une forme en S.

Des chambres de raccordement 27 sont prévues pour chacun des dix tubes de prélèvement 18, suivant la périphérie externe de la bride 17, chacune des chambres 27 étant fermée par un bouchon.

La partie cintrée de direction radiale d'un tube 18 et le prolongement 18' en forme d'S permettent d'assurer le raccordement des tubes de prélèvement 18 entourant le tube central 15 suivant une ligne circulaire de faible diamètre, aux corps 24' des vannes 24 du dispositif de prélèvement 22 disposés suivant une zone circulaire dont le diamètre est supérieur au diamètre de la bride 17.

Comme il est visible sur la figure 6, le dispositif de prélèvement 22 comporte un corps de forme prismatique droite 28 sur lequel est fixée, dans une disposition coaxiale, la rallonge 21 solidaire d'un bouchon 29 destiné à être engagé et fixé dans la cavité usinée sur la seconde face de la bride 17. On assure ainsi le raccordement entre le dispositif de prélèvement 22 et la bride 17.

Le dispositif de prélèvement 22 comporte dix vannes 24 dont les corps 24' sont fixés l'un à la suite de l'autre dans la direction circonférentielle, autour de la bride 28 qui présente de préférence une forme prismatique et une section décagonale.

Sur sa face opposée à la face reliée à la rallonge 21 de raccordement à la bride 17, le corps 28 du dispositif de prélèvement 22 comporte une cavité 30 partiellement obturée suivant la face externe du corps 28, par un couvercle annulaire 31 dans l'alésage interne duquel est fixé de manière étanche un tube 32 communiquant avec un raccord 33 du dispositif de prélèvement. La cavité 30 présente un volume le plus faible possible pour réduire l'inertie du système de prélèvement et limiter les risques de dilution du mélange et constitue, avec l'espace intérieur du tube 32, une chambre de prélèvement 34.

Chacun des corps de vanne 24' des vannes 24 est relié par un tube de raccordement 18'' à la chambre de prélèvement 34. Chaque tube de raccordement 18'' de forme cintrée est relié, à l'une de ses extrémités, au corps 24' d'une vanne 24 et, à sa seconde extrémité, au couvercle 31 de fermeture de la cavité 30, au niveau d'une ouverture traversante. Chacun des corps de vanne 24' d'une vanne 24 comporte une première chambre à laquelle est relié un tube prolongateur 18' et une seconde chambre à laquelle est relié un tube de raccordement 18''. Lorsque la vanne 24 est fermée les deux chambres sont séparées par l'obturateur de la vanne qui peut être une vanne à soufflet. A l'ouverture de la vanne 24, on met en communication entre elles les deux chambres du corps de vanne et le tube de prélèvement 18 avec la chambre de prélèvement 34 par l'intermédiaire des tubes de raccordement 18'et 18''.

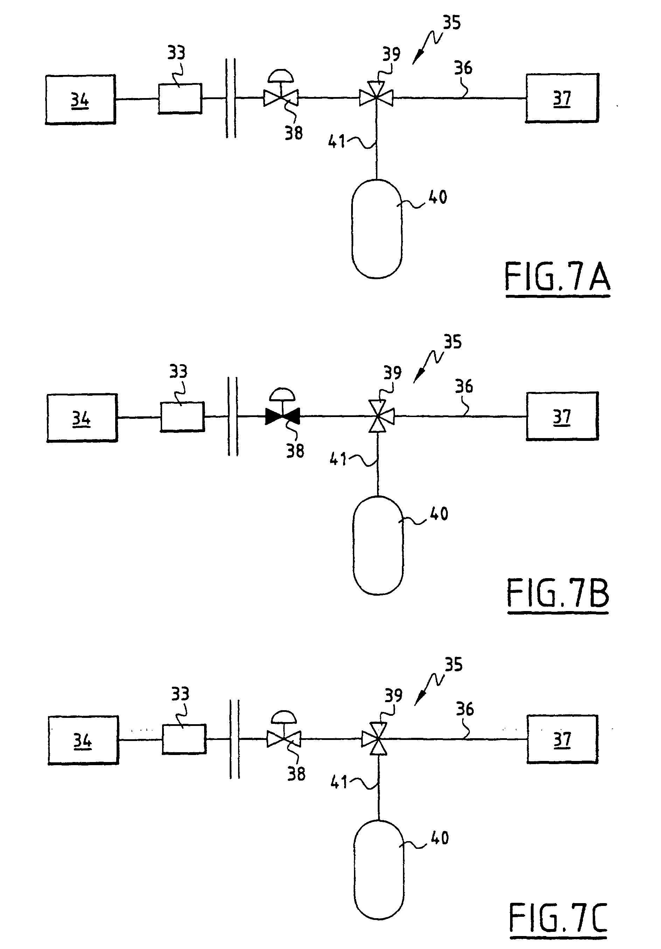

Comme il est visible sur les figures 7A, 7B et 7C, la chambre de prélèvement 34 est reliée par l'intermédiaire du raccord 33 à un circuit 35 de purge, d'analyse et d'injection de gaz.

Le circuit 35 comporte au moins une conduite de liaison 36 entre le raccord 33 de la chambre de prélèvement 34 et au moins un analyseur de gaz 37, par l'intermédiaire d'une vanne d'arrêt 38 et d'une vanne trois voies 39.

La vanne trois voies 39, dont une première voie est reliée à la chambre de prélèvement 34 par l'intermédiaire du raccord 33 et une seconde voie à l'analyseur 37, par la conduite 36, permet, dans une première position représentée sur la figure 7A; de mettre en communication la chambre de prélèvement 34 avec l'analyseur de gaz 37, lorsque la vanne d'isolement 38 est ouverte.

La conduite principale de prélèvement 36 est reliée, par l'intermédiaire de la troisième voie de la vanne trois voies 39, à une conduite en dérivation 41 reliée à un réservoir 40 pouvant renfermer un gaz de purge inerte tel que l'azote, l'argon ou l'hélium ou un gaz réactif tel que l'hydrogène.

Comme il est visible sur la figure 7B, lorsque la vanne trois voies 39 est placée dans sa position représentée et que la vanne 38 est fermée, on peut réaliser la purge de l'analyseur 37, avec le gaz de purge du réservoir 40.

Lorsque la vanne trois voies 39 est placée dans sa position représentée sur la figure 7C et que la vanne d'arrêt 38 est ouverte, on peut envoyer, par l'intermédiaire de la chambre de prélèvement 34, un gaz de purge dans l'un au moins des tubes de prélèvement 18.

Lorsque le réservoir 40 est un réservoir de gaz actif tel que l'hydrogène, on peut envoyer le gaz actif dans au moins un tube de prélèvement 18 par l'intermédiaire de la chambre de prélèvement 34, la vanne trois voies 39 étant dans sa position représentée sur la figure 7C et la vanne d'arrêt 38 étant ouverte.

Par actionnement des vannes 24 du dispositif de prélèvement et de distribution 22 qui sont de préférence des électrovannes et des vannes du circuit 35, on peut donc réaliser la purge de l'analyseur de gaz, la purge d'un tube de prélèvement 18 quelconque ou de plusieurs tubes de prélèvement, ou encore l'injection de gaz actifs tels que l'hydrogène dans le four tournant de l'installation de conversion, au niveau d'un point de référence 14 quelconque ou de plusieurs points de référence de l'enceinte du four.

Comme il sera expliqué ci-après, le dispositif qui a été décrit permet de réaliser, dans de très bonnes conditions, le prélèvement d'échantillons de gaz en une pluralité de points répartis suivant la direction longitudinale du four et l'analyse des échantillons gazeux, ce qui permet d'obtenir une courbe d'évolution des réactions chimiques dans le four et donc une modélisation du four de production d'oxyde d'uranium.

A partir d'une modélisation du four de production d'oxyde, on peut déterminer les injections de gaz réactifs et en particulier d'hydrogène nécessaires pour obtenir, en sortie du four, un oxyde d'uranium de composition moyenne $UO_{2+x}$ dont le rapport O/U est à une valeur souhaitée.

L'injection de gaz réactifs peut être réalisée en un ou plusieurs points de référence de l'enceinte du four correspondant chacun à un point de prélèvement 14 en ouvrant une ou plusieurs vannes 24 du dispositif de prélèvement et d'injection 22.

Il est possible également d'injecter du gaz réactif en chacun des points de référence du four tournant en ouvrant toutes les vannes 24.

Pour la mise en oeuvre de la phase de prélèvement et d'analyse de gaz dans le four tournant, on réalise tout d'abord une purge de l'analyseur 37 par un gaz inerte puis une purge d'un premier conduit de prélèvement. On réalise ensuite le prélèvement de gaz réactif au point de référence 14 correspondant du four par le tube de prélèvement dont on a réalisé précédemment la purge. Les gaz prélevés sont envoyés dans l'analyseur de gaz 37 qui fournit la composition du mélange gazeux réactif prélevé, c'est-à-dire la concentration ou la pression partielle de gaz tels que l'acide fluorhydrique HF et/ou la vapeur d'eau dans le mélange et/ou l'hydrogène et/ou l'azote.

Pour réaliser une analyse sur un prélèvement parfaitement représentatif de l'atmosphère du four dans la zone de prélèvement considérée, il est nécessaire de s'assurer que le mélange gazeux n'est pas modifié par une réaction interne par dépôt de substances ou condensation de gaz prélevés.

Pour éviter l'évolution d'une réaction chimique dans le prélèvement de gaz entre le point de prélèvement et l'analyseur, on réalise un filtrage du mélange gazeux à l'entrée du tube de prélèvement, en le faisant passer à travers un filtre métallique en un matériau résistant à l'atmosphère du four, par exemple un alliage de nickel, de manière à arrêter les particules de matière solide qui pourraient se trouver en suspension dans l'échantillon de gaz prélevé. On évite ainsi le développement de la réaction chimique par suppression d'un composant.

Pour éviter toute condensation ou dépôt dans l'échantillon gazeux prélevé, on réalise un chauffage des parties des tubes de prélèvement situées à l'extérieur du four et raccordées à l'analyseur. On réalise également un chauffage des vannes 24 du dispositif de prélèvement 22.

Il est possible d'utiliser un seul analyseur et donc une seule ligne de circulation des échantillons gazeux prélevés entre le dispositif de prélèvement 22 et l'analyseur 37 ou, au contraire, plusieurs analyseurs reliés au dispositif de prélèvement 22, chacun des analyseurs permettant par exemple de doser l'un des gaz dans le mélange gazeux réactif prélevé dans le four.

Le procédé et le dispositif suivant l'invention permettent donc de réaliser, dans une première phase de modélisation du four, des prélèvements d'échantillons gazeux dans le four et des analyses de ces prélèvement gazeux, de telle manière que le résultat des analyses soit tout à fait représentatif de la composition du mélange gazeux réactif prélevé aux points de prélèvement à l'intérieur du four.

On peut donc obtenir une modélisation très précise du four tournant de conversion par détermination de l'état d'avancement des réactions chimiques de conversion en chacun des points de prélèvement du four et plus particulièrement, l'état d'achèvement de ces réactions chimiques. La modélisation est obtenue sous la forme de courbes d'évolution des réactions chimiques de formation des oxydes suivant la direction longitudinale du four.

Par exemple, on peut effectuer un suivi de la concentration de HF ou $H_2$ dans le four, suivant sa direction longitudinale et en déduire l'état d'avancement des réactions de la transformation de l'oxyfluorure d'uranium.

On peut, à partir de la modélisation du four, déterminer de manière précise les points de référence de l'enceinte du four où doivent être réalisées des injections de gaz réactif tel que l'hydrogène et le débit de gaz pour piloter les réactions chimiques de manière à obtenir, en sortie du four, un oxyde d'uranium d'une composition moyenne souhaitée.

Cette composition moyenne est alors obtenue sans réaliser le mélange de poudres d'oxydes de compositions différentes, la poudre sortant du four présentant la composition désirée.

En outre, l'injection de gaz réactifs, directement dans une zone du four où ces gaz réactifs sont nécessaires, permet d'éviter une déperdition des gaz réactifs qui est un inconvénient lors de l'injection des gaz réactifs par l'extrémité du four tournant. Les gaz réactifs ne sont pas dilués par l'atmosphère du four et sont introduits à l'endroit précis où ils doivent être utilisés, ce qui limite les quantités de gaz réactifs utilisés. On abaisse donc ainsi le coût de la production des oxydes d'uranium.

Le prélèvement de gaz dans le four est réalisé sans utiliser de moyens d'aspiration ou de pompage, du fait que le four est en surpression par rapport à l'atmosphère extérieure. Lorsqu'on ouvre une vanne du dispositif de prélèvement, le gaz réactif s'écoule donc à grande vitesse dans le tube de prélèvement 18 dont la section est suffisamment faible pour que le débit de gaz prélevé reste très limité. On obtient de ce fait des échantillons prélevés dont la composition est tout à fait représentative de la composition de l'atmosphère du four aux points de prélèvement.

Dans le cas de la conversion de l'oxyfluorure d'uranium en oxyde d'uranium, le dosage de l'hydrogène dans les prélèvements permet en particulier de déterminer où et dans quelle mesure se produit la formation d'oxyde $UO_2$ par réduction. On peut ainsi agir sur les réactions pour déplacer le point de transformation totale en $UO_2$, de manière à modifier la composition des oxydes et le rapport O/U.

Le dosage de l'acide fluorhydrique HF dans les prélèvements permet de suivre la transformation de l'oxyfluorure d'uranium en oxyde.

L'invention ne se limite pas au mode de réalisation qui a été décrit.

C'est ainsi qu'on peut réaliser les prélèvements et l'injection de gaz dans le four par des moyens différents de ceux qui ont été décrits.

On peut réaliser le prélèvement d'échantillons de l'atmosphère du four et l'injection de gaz en un nombre de points quelconque. Par exemple, on peut mettre en oeuvre l'invention de manière avantageuse dans un four de conversion d'oxyfluorure d'uranium, en réalisant une injection en cinq points sur les dix points de prélèvement.

Bien entendu, la nature et le débit des gaz injectés dépendent de la nature des réactions chimiques se produisant dans le four et des débits de matière en circulation.

L'invention ne se limite pas aux fours de conversion d'oxyfluorure d'uranium en oxyde d'uranium mais peut connaître des applications dans de nombreux fours dans lesquels circule une matière sous forme dense, par exemple une matière pulvérulente, pâteuse ou même liquide, qui entre en contact avec un mélange gazeux réactif et subit de ce fait, par réaction chimique, des transformations dans le four.

L'invention peut également s'appliquer en dehors du domaine de l'élaboration de poudres pour la fabrication de combustible nucléaire.

## Revendications

1. Procédé de détermination de l'évolution de la conversion d'oxyfluorure d'uranium ($UO_2F_2$) sous forme pulvérulente en circulation dans un four tournant (4, 5), en contact avec un mélange gazeux réactif réalisant la conversion de l'oxyflurorure d'uranium en oxydes d'uranium sous forme pulvérulente par des réactions chimiques se produisant pendant la circulation des produits sous forme pulvérulente à l'intérieur du four, de sorte que la composition des produits sous forme pulvérulente et la composition du mélange gazeux renfermant en particulier $H_2O$, $H_2$ et HF, en circulation dans le four évoluent suivant la direction longitudinale du four, **caractérisé par le fait qu'**on prélève une pluralité d'échantillons du mélange gazeux chacun en un point d'une pluralité de points de référence (14) espacés les uns des autres suivant la direction longitudinale (6) du four (4) pour déterminer la composition du mélange gazeux, qu'on déduit de la composition du mélange gazeux en chacun des points de référence le degré d'avancement des réactions chimiques de formation des oxydes d'uranium dans le four tournant, suivant sa direction longitudinale, et qu'on réalise une modélisation du fonctionnement du four tournant, sous la forme de courbes d'évolution des réactions chimiques de formation des oxydes d'uranium suivant la direction longitudinale (6) du four tournant (4).

2. Procédé de réglage de la conversion d'oxyfluorure d'uranium ($UO_2F_2$) sous forme pulvérulente circulant dans la direction longitudinale (6) d'un four tournant (4) en contact avec un mélange gazeux réactif renfermant en particulier de l'hydrogène et de la vapeur d'eau, de manière à convertir l'oxyfluorure d'uranium en oxydes d'uranium, **caractérisé par le fait qu'**on réalise; préalablement, une modélisation du four tournant (4) par le procédé suivant la revendication 1, et qu'on injecte dans le four tournant (4), en au moins un point de référence (14) défini à partir de la modélisation du four tournant (4, 5) au moins un gaz réactif tel que l'hydrogène défini quant à sa nature et son débit par la modélisation du four, de manière à obtenir à la sortie du four tournant (4, 5) des oxydes d'uranium ayant une composition moyenne ($UO_{2+x}$) et un rapport O/U prédéterminé.

3. Procédé suivant la revendication 2, **caractérisé par le fait que** le rapport O/U des oxydes d'uranium à la sortie du four tournant (4, 5) prédéterminé est compris entre 2 et 2,7.

4. Procédé suivant la revendication 3, **caractérisé par le fait que** le rapport O/U des oxydes d'uranium à la sortie du four tournant prédéterminé est compris entre 2,10 et 2,25.

5. Procédé suivant la revendication 1 ou l'une quelconque des revendications 2 à 4, **caractérisé par le fait qu'**on détermine la concentration d'acide fluorhydrique HF dans les échantillons gazeux prélevés dans le four tournant (4, 5).

6. Procédé suivant la revendication 1, ou l'une quelconque des revendications 2 à 5, **caractérisé par le fait qu'**on détermine, dans les échantillons gazeux prélevés dans le four tournant (4, 5), la concentration de l'un au moins des gaz hydrogène, azote et vapeur d'eau.

7. Dispositif pour la mise en oeuvre d'un procédé selon l'une quelconque des revendications 1 à 6, **caractérisé par le fait qu'**il comporte une canne de prélèvement et d'injection (10) comprenant un tube central de support (15), des moyens (17) de fixation du tube central (15) à l'intérieur du four (4, 5), dans une disposition parallèle à la direction longitudinale (6) du four (4, 5), une pluralité de tubes de prélèvement (18) parallèles à l'axe du tube central (15) et répartis suivant la périphérie du tube central (15) et un dispositif de prélèvement (22) disposé à l'extérieur du four (4, 5) relié à au moins un analyseur de gaz (37), chacun des tubes de prélèvement (18) comportant une première extrémité débouchant dans le four en un point de prélèvement (14) et une seconde extrémité reliée au dispositif de prélèvement (22) qui comporte des vannes (24) pour relier successivement ou de manière groupée chacun des tubes de prélèvement (18) à l'au moins un analyseur (37).

8. Dispositif suivant la revendication 7, **caractérisé par le fait qu'**une pluralité d'éléments de support et de guidage (16, 16') de forme générale cylindrique sont fixés dans une disposition coaxiale autour du tube central (15) en des emplacements espacés suivant la direction axiale du tube central (15), chaque élément de support et de guidage (16, 16') comportant une pluralité de logements (26) destinés chacun à un tube de prélèvement (18) de la pluralité de tubes de prélèvement (18).

9. Dispositif suivant l'une quelconque des revendications 7 et 8, **caractérisé par le fait que** le dispositif de prélèvement (22) disposé à l'extérieur du four (4, 5) comporte un corps (28) relié au tube central (15) de la canne de prélèvement (10) par l'intermédiaire de l'élément (17) de fixation de la canne de prélèvement (10) à l'intérieur du four (4, 5), une chambre de prélèvement (34) étant ménagée dans le corps (28) du dispositif de prélèvement et une pluralité de vannes (24, 24') réparties autour du corps (28) du dispositif de prélèvement (22) et comportant chacune une chambre (24') en deux parties séparées l'une de l'autre par un obturateur de la vanne (24), les tubes de prélèvement (18, 18') étant reliés, à leur seconde extrémité, chacun à une première chambre d'un corps de vanne (24') et un tube de raccordement (18") reliant la seconde chambre du corps de vanne (24') à la chambre de prélèvement (34) qui est elle-même reliée à l'au moins un analyseur (37) par l'intermédiaire d'au moins un conduit (26) sur lequel est placée au moins une vanne (38, 39).

10. Dispositif suivant la revendication 9, **caractérisé par le fait que** la chambre de prélèvement (34) du dispositif de prélèvement (22) est reliée à l'au moins un analyseur (37), par l'intermédiaire d'une conduite (36) sur laquelle sont disposées successivement, entre la chambre (34) et l'au moins un analyseur (37), une vanne d'arrêt (38) et une vanne trois voies (39) dont une première voie est reliée à la partie de la conduite (36) communiquant avec la chambre de prélèvement (34), une seconde voie à la partie de la conduite (36) reliée à l'au moins un analyseur (37) et une troisième voie à un réservoir (40) renfermant l'un des gaz suivants : gaz de purge inerte tels que l'azote, l'argon ou l'hélium, gaz réactif tel que l'hydrogène.

11. Dispositif suivant l'une quelconque des revendications 7 à 10, **caractérisé par le fait qu'**à l'extrémité de chacun des tubes de prélèvement (18) est placé un filtre destiné à arrêter le produit à état condensé tel qu'un solide pulvérulent pour éviter une évolution de l'au moins une réaction chimique à l'intérieur du tube de prélèvement (18) entre le point de prélèvement (14) et l'au moins un analyseur (37).

12. Dispositif suivant l'une quelconque des revendications 7 à 11, **caractérisé par le fait que** les tubes de prélèvement (18) et les vannes (24) du dispositif de prélèvement (22) sont chauffés pour éviter une condensation de produit gazeux dans les tubes de prélèvement (18) entre les points de prélèvement (14) et l'au moins un analyseur (37).

13. Dispositif suivant l'une quelconque des revendications 7 à 12, **caractérisé par le fait que** l'élément (17) de fixation du tube central (15) de la canne de prélèvement (10) est constitué par une bride comportant des moyens de passage et de raccordement au dispositif de prélèvement (22) de chacun des tubes (18).

14. Dispositif suivant l'une quelconque des revendications 7 à 13, **caractérisé par le fait que** la canne de prélèvement (10) est fixée à une extrémité d'un four tournant (4, 5) de conversion d'oxyfluorure d'uranium en oxydes d'uranium, suivant la direction axiale (6) du four tournant.

**Patentansprüche**

1. Verfahren zur Bestimmung des Fortschreitens der Umwandlung von pulverförmigem Uranoxyfluorid ($UO_2F_2$), das in einem Trommelofen (4, 5) zirkuliert, im Kontakt mit einer reaktionsfähigen Gasmischung, welche die Umwandlung von Uranoxyfluorid in pulverförmige Uranoxide bewirkt durch chemische Reaktionen, die während der Zirkulation der pulverförmigen Produkte im Innern des Ofens ablaufen, in der Weise, dass die Zusammensetzung der pulverförmigen Produkte und die Zusammensetzung der Gasmischung, die insbesondere $H_2O$, $H_2$ und HF enthält, bei der Zirkulation in dem Ofen entlang der Längsrichtung des Ofens sich ändern, **dadurch gekennzeichnet, dass** man an einem Punkt einer Vielzahl von Bezugs- bzw. Entnahmepunkten (14), die im Abstand voneinander entlang der Längsrichtung (6) des Ofens (4) angeordnet sind, eine Vielzahl von Proben jeder Gasmischung entnimmt, um die Zusammensetzung der Gasmischung zu bestimmen, dass man aus der Zusammensetzung der Gasmischung an jedem der Entnahmepunkte den Grad des Fortschreitens der chemischen Reaktionen zur Bildung von Uranoxiden in dem Trommelofen entlang der Längsrichtung desselben ableitet, und dass man daraus ein Modell der Funktionsweise des Trommelofens in Form von Kurven erstellt, die das Fortschreiten der chemischen Reaktionen zur Bildung von Uranoxiden entlang der Längsrichtung (6) des Trommelofens (4) anzeigen.

2. Verfahren zur Einstellung der Umwandlung von pulverförmigem Uranoxyfluorid ($UO_2F_2$), das in der Längsrichtung (6) eines Trommelofens (4) im Kontakt mit einer reaktionsfähigen Gasmischung zirkuliert, die insbesondere Wasserstoff und Wasserdampf enthält, um so Uranoxyfluorid in Uranoxide umzuwandeln, **dadurch gekennzeichnet, dass** man vorher unter Anwendung des Verfahrens nach Anspruch 1 ein Modell des Trommelofens (4) erstellt, und dass man in den Trommelofen (4) an mindestens einem Bezugspunkt (14), der anhand des Modells des Trommelofens (4, 5) definiert wird, mindestens ein reaktionsfähiges Gas wie Wasserstoff, das hinsichtlich seiner Art und seiner Zuführungsmenge durch das Ofenmodell definiert wird, so einleitet, dass am Auslass des Trommelofens (4, 5) Uranoxide mit einer mittleren Zusammensetzung ($UO_{2+x}$) und einem vorgegebenen O/U-Verhältnis erhalten werden.

3. Verfahren nach Anspruch 2, **dadurch gekennzeichnet, dass** das vorgegebene O/U-Verhältnis der Uranoxide am Auslass des Trommelofens (4, 5) zwischen 2 und 2,7 liegt.

4. Verfahren nach Anspruch 3, **dadurch gekennzeichnet, dass** das vorgegebene O/U-Verhältnis der Uranoxide am Auslass des Trommelofens zwischen 2,10 und 2,25 liegt.

5. Verfahren nach Anspruch 1 oder einem der Ansprüche 2 bis 4, **dadurch gekennzeichnet, dass** man die Fluorwasserstoffsäure (HF)-Konzentration in den aus dem Trommelofen (4, 5) entnommenen Proben bestimmt.

6. Verfahren nach Anspruch 1 oder einem der Ansprüche 2 bis 5, **dadurch gekennzeichnet, dass** man in den aus dem Trommelofen (4, 5) entnommenen gasförmigen Proben die Konzentration mindestens eines der Gase aus der Gruppe Wasserstoff, Stickstoff und Wasserdampf bestimmt.

7. Vorrichtung zur Durchführung eines Verfahrens nach einem der Ansprüche 1 bis 6, **dadurch gekennzeichnet, dass** sie einen Entnahme- und Einleitungsstab (10) umfasst, der ein zentrales Trägerrohr (15), Einrichtungen (17) zur Fixierung des zentralen Trägerrohrs (15) im Innern des Ofens (4, 5) in einer Anordnung parallel zur Längsrichtung (6) des Ofens (4, 5), eine Vielzahl von Entnahmerohren (18) parallel zur Achse des zentralen Rohrs (15), die über den Umfang des zentralen Rohrs (15) verteilt sind, und eine Entnahmeeinrichtung (22), die im Innern des Ofens (4, 5) angeordnet und mit mindestens einem Gas-Analysator (37) verbunden ist, umfasst, wobei jedes der Entnahmerohre (18) umfasst ein erstes Ende, das an einem Entnahmepunkt (14) in den Ofen mündet, und ein zweites Ende, das mit der Entnahmeeinrichtung (22) verbunden ist, die Ventile (24) aufweist, um nacheinander oder in gruppenförmiger Anordnung jedes der Entnahmerohre (18) mit mindestens einem Analysator (37) zu verbinden.

8. Vorrichtung nach Anspruch 7, **dadurch gekennzeichnet, dass** eine Vielzahl von Träger- und Führungselementen (16, 16') mit einer im Allgemeinen zylindrischen Form in einer coaxialen Anordnung um das zentrale Rohr (15) herum an Stellen fixiert sind, die im Abstand voneinander entlang der Längsachse des zentralen Rohres (15) angeordnet sind, wobei jedes Träger- und Führungselement (16, 16') eine Vielzahl von Ausnehmungen (26) aufweist, die jeweils bestimmt sind für ein Entnahmerohr (18) der Vielzahl von Entnahmerohren (18).

9. Vorrichtung nach einem der Ansprüche 7 und 8, **dadurch gekennzeichnet, dass** die Entnahmeeinrichtung (22), die außerhalb des Ofens (4, 5) angeordnet ist, umfasst einen Körper (28), der mit dem zentralen Rohr (15) des Entnahmestabes (10) über das Element (17) zur Fixierung des Entnahmestabes (10) im Innern des Ofens (4, 5) verbunden ist, eine Entnahmekammer (34), die in dem Körper (28) der Entnahmeeinrichtung vorgesehen ist, und eine Vielzahl von Ventilen (24, 24'), die um den Körper (28) der Entnahmeeinrichtung (22) herum verteilt sind und jeweils eine in zwei Teile unterteilte Kammer (24') aufweisen, die durch einen Ventilschieber (24) voneinander getrennt sind, wobei die Entnahmerohre (18, 18') an ihrem zweiten Ende jeweils mit einer ersten Kammer eines Ventilkörpers (24') und einem Verbindungsrohr (18") verbunden sind, welches die zweite Kammer des Ventilkörpers (24') mit der Entnahmekammer (34) verbindet, die ihrerseits über mindestens eine Rohrleitung (26), auf der mindestens ein Ventil (38, 39) angeordnet ist, mit mindestens einem Analysator (37) verbunden ist.

10. Vorrichtung nach Anspruch 9, **dadurch gekennzeichnet, dass** die Entnahmekammer (34) der Entnahmeeinrichtung (22) mit mindestens einem Analysator (37) verbunden ist über eine Rohrleitung (36), auf der zwischen der Kammer (34) und mindestens einem Analysator (37) nacheinander angeordnet sind ein Absperr-Ventil (38) und ein Drei-Wege-Ventil (39), dessen erster Abgang mit dem Abschnitt der Rohrleitung (36) verbunden ist, der mit der Entnahmekammer (34) in Verbindung steht, dessen zweiter Abgang mit dem Abschnitt der Rohrleitung (36) verbunden ist, der mit mindestens einem Analysator (37) in Verbindung steht, und dessen dritter Abgang mit einem Reservoir (40) verbunden ist, das eines der folgenden Gase enthält: ein inertes Spülgas wie Stickstoff, Argon oder Helium, ein reaktionsfähiges Gas wie Wasserstoff.

11. Vorrichtung nach einem der Ansprüche 7 bis 10, **dadurch gekennzeichnet, dass** am Ende jedes der Entnahmerohre (18) ein Filter angeordnet ist, der dazu bestimmt ist, das Produkt im kondensierten Zustand, wie z.B. einen pulverförmigen Feststoff, zu blockieren, um ein Fortschreiten der mindestens einen chemischen Reaktion im Innern des Entnahmerohres (18) zwischen dem Entnahmepunkt (14) und dem mindestens einen Analysator (37) zu verhindern.

12. Vorrichtung nach einem der Ansprüche 7 bis 11, **dadurch gekennzeichnet, dass** die Entnahmerohre (18) und die Ventile (24) der Entnahmeeinrichtung (22) erwärmt werden, um eine Kondensation des gasförmigen Produkts in den Entnahmerohren (18) zwischen den Entnahmepunkten (14) und dem mindestens einen Analysator (37) zu verhindern.

13. Vorrichtung nach einem der Ansprüche 7 bis 12, **dadurch gekennzeichnet, dass** das Element (17) zur Fixierung des zentralen Rohrs (15) des Entnahmestabes (10) aus einem Flansch besteht, der Einrichtungen zum Hindurchführen und Verbinden jedes der Rohre (18) mit der Entnahmeeinrichtung (22) aufweist.

14. Vorrichtung nach einem der Ansprüche 7 bis 13, **dadurch gekennzeichnet, dass** der Entnahmestab (10) an einem Ende eines Trommelofens (4, 5) zur Umwandlung von Uranoxyfluorid in Uranoxide entlang der axialen Richtung (6) des Trommelofens fixiert ist.

## Claims

1. Method for determining the evolution of the conversion of pulverulent uranium oxyfluoride (UO$_2$F$_2$) circulating in a rotary kiln (4, 5), in contact with a reactive gas mixture, performing the conversion of uranium oxyfluoride into pulverulent uranium oxides by chemical reactions occurring during the circulation of the pulverulent products within the kiln, in such a way that the composition of the pulverulent products and the composition of the gas mixture more particularly containing H$_2$O, H$_2$ and HF, circulating in the kiln in the longitudinal direction thereof, **characterized in that** a plurality of samples of the gas mixture are taken, each at one of a plurality of reference points (14) spaced from one another in the longitudinal direction (6) of the kiln (4) in order to determine the composition of the gas mixture, that from the composition of the gas mixture at each of the reference points deduction takes place of the degree of progress of the chemical uranium oxide formation reactions in the rotary kiln in the longitudinal direction thereof and that modelling takes place of the operation of the rotary kiln, in the form of evolution curves of the chemical uranium oxide formation reactions in the longitudinal direction (6) of the rotary kiln (4).

2. Method for adjusting the conversion of pulverulent uranium oxyfluoride (UO$_2$F$_2$) circulating in the longitudinal direction (6) of a rotary kiln (4) in contact with a reactive gas mixture more particularly containing hydrogen and steam, in such a way as to convert the uranium oxyfluoride into uranium oxides, **characterized in that** beforehand there is a modelling of the rotary kiln (4) by the method according to claim 1, and that injection takes place into the rotary kiln (4) at at least one reference point (14) defined on the basis of the modelling of the rotary kiln (4, 5), of at least one reactive gas such as hydrogen, whose nature and flow rate are defined by the kiln modelling, so as to obtain at the outlet of the rotary kiln (4, 5) uranium oxides having an average composition (UO$_{2+x}$) and a predetermined O/U ratio.

3. Method according to claim 2, **characterized in that** the predetermined O/U ratio of the uranium oxides at the outlet of the rotary kiln (4, 5) is between 2 and 2.7.

4. Method according to claim 3, **characterized in that** the predetermined O/U ratio of the uranium oxides at the outlet of the rotary kiln is between 2.10 and 2.25.

5. Method according to claim 1 or any one of the claims 2 to 4, **characterized in that** the concentration of hydrofluoric acid (HF) in the gas samples taken in the rotary kiln (4, 5) is determined.

6. Method according to claim 1 or any one of the claims 2 to 5,
**characterized in that** the concentration of at least one of the gases hydrogen, nitrogen and steam in the gas samples taken in the rotary kiln (4, 5) is determined.

7. Device for performing a method according to any one of the claims 1 to 6, **characterized in that** it comprises a sampling and injection rod (10) having a central support tube (15), means (17) for fixing the central tube (15) within the kiln (4, 5), in a position parallel to the longitudinal direction (6) of the kiln (4, 5), a plurality of sampling tubes (18) parallel to the axis of the central tube (15) and distributed along the periphery of the central tube (15) and a sampling device (22) located outside the kiln (4, 5) and connected to at least one gas analyzer (37), each of the sampling tubes (18) having a first end issuing into the kiln at a sampling point (14) and a second end connected to the sampling device (22) having valves (24) for connecting successively or in grouped manner each of the sampling tubes (18) to at least one analyzer (37).

8. Device according to claim 7, **characterized in that** a plurality of generally cylindrical support and guidance elements (16, 16') are fixed in a coaxial arrangement around the central tube (15) at locations spaced in the axial direction of the central tube (15), each support and guidance element (16, 16') having a plurality of recesses (26), each for receiving one of the plurality of sampling tubes (18).

9. Device according to either of the claims 7 and 8, **characterized in that** the sampling device (22) positioned outside the kiln (4, 5) comprises a body (28) connected to the central tube (15) of the sampling rod (10) by means of the element (17) for fixing the sampling rod (10) to the interior of the kiln (4, 5), a sampling chamber (34) being located in the body (28) of the sampling device and a plurality of valves (24, 24') distributed around the body (28) of the sampling device (22) and each having a chamber (24') in two parts separated from one another by obturator of the valve (24), the sampling tubes (18, 18') being connected at their second end to in each case a first chamber of a valve body (24') and a connection tube (18") connecting the second chamber of the body (24') to the sampling chamber (34), which is itself connected to at least one analyzer (37) via at least one duct (26) on which is placed at least one valve (38, 39).

10. Device according to claim 9, **characterized in that** the sampling chamber (34) of the sampling device (22) is connected to at least one analyzer (37) via a duct (36) on which are successively arranged, between the chamber (34) and at least one analyzer (37), a stop valve (38) and a three-way valve (39), whereof a first way is connected to the part of the duct (36) communicating with the sampling chamber (34), a second way to the part of the duct (36) connected to the at least one analyzer (37), and a third way to a tank (40) containing one of the following gases: an inert bleeding gas such as nitrogen, argon or helium, or reactive gas such as hydrogen.

11. Device according to any one of the claims 7 to 10, **characterized in that** at the end of each of the sampling tubes (18) is placed a filter for stopping the product in the condensed state, such as a pulverulent solid, in order to prevent an evolution of at least one chemical reaction within the sampling tube (18) between the sampling point (14) and the at least one analyzer (37).

12. Device according to any one of the claims 7 to 11, **characterized in that** the sampling tubes (18) and valves (24) of the sampling device (22) are heated to prevent condensation of the gaseous product in the sampling tubes (18) between the sampling points (14) and the at least one analyzer (37).

13. Device according to any one of the claims 7 to 12, **characterized in that** the fixing element (17) of the central tube (15) of the sampling rod (10) is constituted by a flange having means for the passage and connection to the sampling device (22) of each of the tubes (18).

14. Device according to any one of the claims 7 to 13, **characterized in that** the sampling rod (10) is fixed to one end of a rotary kiln (4, 5) for converting uranium oxyfluoride into uranium oxides, in the axial direction (6) of said rotary kiln.

2

1

4 7 11

3 5a 13 14 8 5 14 8 14 6 5b 10

33 12

α

FIG.1

FIG.2

FIG.3A

FIG.3B

EP 1 254 076 B1

FIG.5

FIG.4

FIG.6

FIG.7A

FIG.7B

FIG.7C